# EUROPÄISCHE PATENTSCHRIFT

(11) **EP 0 345 562 B1**
(45) Veröffentlichungstag und Bekanntmachung des Hinweises auf die Patenterteilung: **21.04.1993**
(21) Anmeldenummer: 89109514.3
(22) Anmeldetag: 26.05.1989
(51) Int. Cl.: G01N 33/00

(54) **Verfahren und Vorrichtung zur Messwertverarbeitung**
Method and apparatus for processing measuring data
Procédé et appareil pour le traitement de données de mesures

(30) Priorität: 04.06.1988 DE 3819101
(43) Veröffentlichungstag der Anmeldung: 13.12.1989
(73) Patentinhaber: Conducta Gesellschaft für Mess- und Regeltechnik mbH & Co., D-70839 Gerlingen (DE)
(72) Erfinder: Ambos, Stefan, D-7050 Waiblingen (DE)
(74) Vertreter: Otte, Peter, Dipl.-Ing.

(56) Entgegenhaltungen:
- DE-A- 2 946 775
- FR-A- 1 375 183
- US-A- 4 151 738
- PATENT ABSTRACTS OF JAPAN vol. 9, no. 151 (P-367)(1874) 26 Juni 85, JP-A-60 27849

## Beschreibung

### Stand der Technik

In der Gasanalyse werden als Sensoren auch zur Konzentrationsbestimmung von Gasen, im allgemeineren Sinn aber auch zur Meldung von Gefahren beim Arbeiten mit Mikrowellen, bei der Rauch- und Feuerdetektion u.dgl. Sensorelemente eingesetzt, die als Halbleiter oder als speziell aufbereitete Widerstände ihren Widerstandswert in Abhängigkeit von der zu messenden Größe, speziell also einer zu messenden Gaskonzentration ändern. Solche Gasanalysesysteme sind z.B. aus US-A-4 151 738 bekannt.

Bei solchen bekannten Verfahren zur Meßwertverarbeitung, speziell also beim Einsatz von Halbleitersensoren als Maß für eine zu messende Gaskonzentration und hierauf wird im folgenden genauer eingegangen, weil dies auch das bevorzugte Anwendungsgebiet vorliegender Erfindung ist, die hierauf aber nicht eingeschränkt ist, erfolgt die Aussage über die gemessene Gaskonzentration anhand des sich ändernden Widerstandes, der im folgenden mit RS bezeichnet wird. Allerdings ist ein solcher, seinen Widerstandswert in Abhängigkeit zur messenden Größe verändernder Sensor nicht in der Lage, etwa bei der Konzentration Null ebenfalls mit dem Widerstandswert Null zu beginnen, sondern der jeweilige Halbleitersensor verfügt über einen Basis- oder Grundwiderstandswert Ro, der zunächst als konstant angesehen werden soll, allerdings nur mit Bezug auf die zu messende Gaskonzentration, da dieser Grundwiderstand mannigfaltige andere Abhängigkeiten aufweist und vielen, zur eigentlichen Messung fremden Einflüssen unterworfen sein kann.

Was daher im Normalfall gemessen wird, ist die Summe des Grundwiderstandswerts Ro zuzüglich dem Widerstandsanteil ΔRS als der sich bei der Messung jeweils ergebende Gesamtwiderstand Rs.

Der Grundwiderstandswert Ro schwankt dabei produktionstechnisch für bestimmte Sensortypen zwischen 5 kOhm und 15 kOhm und ist bzw. kann neben diesen starken Exemplarsteuerungen ferner noch abhängig sein von Luftfeuchtigkeit, Anströmungsgeschwindigkeit und Winkel, Alterung, Langzeitstabilität und weist einen unter Umständen erheblichen Temperaturgang auf. Diese Parameter gehen bei der Auswertung von Meßergebnissen unter Zugrundelegung des vom Halbleitersensor jeweils eingenommenen Gesamtwiderstandswerts, wenn man beispielsweise sich ändernde Gaskonzentrationen mißt, als Meßfehler in das Ergebnis ein bzw. müssen (nachträglich) umständlich korrigiert und berücksichtigt werden. Hierzu ist es häufig notwendig, mindestens noch die Temperatur am Meßort zu erfassen und bei der Auswertung des Widerstands-Meßergebnisses zu berücksichtigen.

Der Erfindung liegt daher die Aufgabe zugrunde, bei einem Meßverfahren und einer Vorrichtung zur Meßwertverarbeitung dafür zu sorgen, daß zusätzliche Abhängigkeiten des gewonnenen Meßwerts bei einer Messung mit einem seinen Widerstandswert ändernden Sensor minimiert werden bzw. vollständig entfallen.

### Vorteile der Erfindung

Die Erfindung löst diese Aufgabe mit den kennzeichnenden Merkmalen des Hauptanspruchs bzw. des Anspruchs 4 und hat den Vorteil, daß es möglich ist, auch mit preisgünstigen, daher im Grundwiderstandswert auch stark streuenden (Halbleiter)Sensoren sehr genaue Meßergebnisse zu erhalten, ohne daß die auf den Grundwiderstandswert zurückzuführenden und hauptsächlich an diesem auftretenden, unerwünschten Einflußgrößen eine nennenswerte Rolle spielen.

Der Erfindung gelingt daher die Beseitigung der am Basiswiderstand Ro auftretenden verschiedenen Abhängigkeiten, wobei Ro gerade der Widerstandswert ist, der von der zu messenden Gaskonzentration nicht beeinflußt wird. Die Erfindung eliminiert Exemplarstreuungen, Produktionstoleranzen sowie im Verlauf der Messungen über der Zeit entstehende, also alterungsbedingte Abweichungen; alles Störgrößen, die sich im Endeffekt bei der Messung mit dem reinen Widerstandswert RS als Fehler im Meßergebnis wiederfinden.

Vorteilhaft ist ferner, daß bei der (wiederholten) Kontrolle des Ro-Widerstandswerts, die in einem vorgegegebenen Kalibrierhythmus beispielsweise durchgeführt werden kann, die Kalibrierung nicht mit dem Gas durchgeführt zu werden braucht, dessen Konzentration zu ermitteln ist, sondern unter Zugrundelegung eines beliebigen Standard-Kalibriergases geringerer Konzentration für alle Meßkomponenten, die durch solche Halbleiter-Sensoren oder auch Sensoren anderer Bauart bestimmt werden können, wie beispielsweise Methan, Propan, Butan u. dgl.

Durch die in den Unteransprüchen aufgeführten Maßnahmen sind vorteilhafte Weiterbildungen und Verbesserungen der Erfindung möglich. Besonders vorteilhaft ist die wiederholte Abspeicherung des Grundwiderstandswerts Ro durch Kalibrierung innerhalb vorgegebener Zeiträume, so daß sich daran anschließend jeweils mit einem neu ermittelten Ro-Widerstandswert gearbeitet werden kann, was auch die Nullpunkt-Stabilität des Meßsystems erheblich verbessert.

### Zeichnung

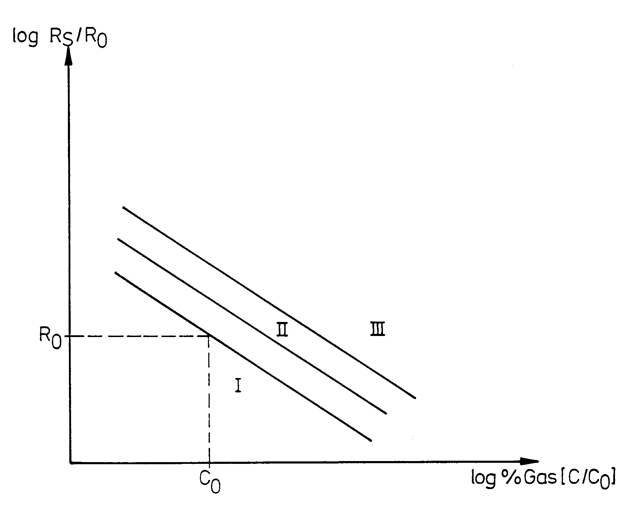

Ein Ausführungsbeispiel der Erfindung ist in der Zeichnung dargestellt und wird in der nachfolgenden Beschreibung näher erläutert.

Die Zeichnung zeigt anhand eines Diagramms die Streuung von Kurvenverläufen von Halbleitersensoren des gleichen Typs mit starken Unterschieden im Grundwiderstandswert aufgrund von Herstellung, Alterung und Umgebungsbedingungen sowie deren Verlauf bei sich ändernder Gaskonzentration.

### Beschreibung der Ausführungsbeispiele

Der Grundgedanke vorliegender Erfindung besteht darin, zur Messung von Gaskonzentrationen zwar weiter den Gesamtwiderstand des eingesetzten (Halbleiter)Sensors zu verwenden - etwas anderes ist ja auch nicht möglich, da der Sensor ein einheitliches Ganzes bildet -; vorher jedoch den Grundwiderstandswert Ro zu bestimmen und der Ausweisung des Meßwerts eine Rechenoperation vorzuschalten, in welcher das Verhältnis des gemessenen Widerstands RS zu dem Grundwiderstandswert Ro ermittelt wird.

In den Kurvenverläufen I, II und II sind die Abhängigkeiten von Signal- oder Meßwiderständen Rs zur Gaskonzentration der jeweils zu messenden Gaskomponente gezeigt, wobei die Unterschiede der parallelen Kurvenverläufe auf die Unterschiede in den absoluten Widerstandswerten von Ro zurückzuführen sind.

Gemäß vorliegender Erfindung wird als Meßwert für eine zu bestimmende Gaskonzentration grundsätzlich der Verhältniswert von Rs/Ro ausgewertet, wobei so vorgegangen wird, daß eine erste (Kalibrier)Messung durchgeführt wird, bei welcher der Halbleitersensor mit Luft oder, was sich als praktisch erwiesen hat, mit einem Standardkalibriergas beliebiger Konzentration zur Ermittlung des Grundwiderstandswerts Ro beaufschlagt wird. Dieses Standard-Kalibriergas kann für beliebige Meßkomponenten verwendet werden, da alterungs- oder umgebungsbedingte Änderungen im Verhältnis Rs/Ro für alle Meßkomponenten eines Sensor gleich sind.

Der so ermittelte Grundwiderstandswert Ro wird gespeichert und allen nachfolgenden Messungen der Gaskonzentration zugrundegelegt, d.h. die sich jeweils ändernden Werte von Rs werden auf den ermittelten Ro-Grundwiderstandswert bezogen.

Es empfiehlt sich, den Grundwiderstandswert Ro in einem vorgegebenen Kalibrierrhythmus von beispielsweise drei Monaten mit dem Standard-Kalibriergas oder Luft automatisch von einem geeigneten Gasdetektionssystem messen zu lassen und als jeweils insofern dann korrigierten neuen Grundwiderstandswert abzuspeichern, wobei durch diese Wiederholung der Ro-Bestimmung in bestimmten Zeitabständen alterungsbedingte Abweichungen entfallen und für den nächsten Kalibrierzeitraum eine einwandfreie Messung sichergestellt ist.

Das bei der Ermittlung des tatsächlichen Meßwerts dann stets zugrundegelegte Verhältnis Rs/Ro bleibt daher (bei konstanter Gaskonzentration) konstant und ist von den weiter vorn genannten störenden Einflußgrößen unabhängig, so daß auch preiswerte Halbleiter-Sensoren in ein zuverlässiges und genaues Meßsystem integriert werden können. Durch die bei jeder Kalibrierung des Gassensors neu ermittelten und für weitere Berechnungen abgespeicherten, jeweils neuen Ro-Werte ergibt sich auch eine wesentlich genauere Nullpunktstabilität.

Dabei hat es sich als vorteilhaft herausgestellt, anstelle von beispielsweise Luft zur Kalibrierung und Bestimmung des Ro-Grundwiderstandswertes ein Standard-Kalibriergas mit einer Konzentration von beispielsweise 3000 ppm Methan (CH₄) zu verwenden, wodurch man einen definitiven Wert in der Ro-Kalibrierung sicherstellt.

Alle in der Beschreibung, den nachfolgenden Ansprüchen und der Zeichnung dargestellten Merkmale können sowohl einzeln als auch in beliebiger Kombination miteinander erfindungswesentlich sein.

## Patentansprüche

1. Verfahren zur Meßwertverarbeitung bei eine Widerstandsänderung für eine zu messende Gaskonzentration auswertende Gasdetektionsvorrichtung, dadurch gekennzeichnet, daß mindestens einmal ein Grundwiderstandswert (Ro) ohne Einfluß des Meßgases ermittelt und gespeichert wird und daß bei jeder sich anschließenden Messung mit der in ihrer Konzentration zu bestimmenden Meßgaskomponente der sich ergebende Gesamtwiderstand (Rs) rechnerisch mit dem Grundwiderstandswert (Ro) ins Verhältnis gesetzt und mit dem gewonnenen Verhältniswert (Rs/Ro) weitergearbeitet wird.

2. Verfahren nach Anspruch 1, dadurch gekennzeichnet, daß der Grundwiderstandswert (Ro) in vorgegebenen Zeitabständen wiederholt gemessen und als jeweils neuer Grundwiderstandswert (Ro) abgespeichert und der Verhältnisbildung zugrundegelegt wird.

3. Verfahren nach Anspruch 1 oder 2, dadurch gekennzeichnet, daß zur wiederholten Messung und späteren Abspeicherung des jeweils neuen Grundwiderstandswerts (Ro) ein Standard-Kalibriergas (3000 ppm CH₄) verwendet wird, wobei nachfolgend ermittelte Gesamtwiderstandswerte (Rs) auf den jeweils neu ermittelten Grundwiderstandswert (Ro) bezogen werden.

4. Vorrichtung zur Durchführung des Verfahrens nach Anspruch 1, dadurch gekennzeichnet, daß ein Speicher vorgesehen ist, in welchem vor der Messung des Gesamtwiderstands des (Halbleiter)Sensors (Rs) der Grundwiderstandswert (Ro) abgespeichert ist, mit Schaltungsmitteln (Mikroprozessor, Kleinrechner, Logikschaltung), die bei jeder neu eingehenden Meldung eines Gesamtwiderstandswertes (Rs) des (Halbleiter)Sensors diesen Wert mit dem jeweils aktuell abgespeicherten Grundwiderstandswert (Ro) ins Verhältnis setzen und zur Weiterverarbeitung ausgeben.

## Claims

1. A method of measured value processing in connection with a change in resistance for a gas detection device which evaluates a gas concentration to be measured**, characterised in that** at least once a basic resistance value (Ro) is determined and stored, without influence from the gas to be measured, and, in connection with every successive measurement in the presence of the measurement-gas components whose concentration is to be determined, the resulting total resistance (Rs) is expressed as a ratio to the basic resistance value (Ro) by calculation, and the resultant ratio (Rs/Ro) is further processed.

2. A method according to claim 1, **characterised in that** the basic resistance value (Ro) is repeatedly measured at given intervals, and is stored as a respective new basic resistance value (Ro), and the ratio established is based thereon.

3. A method according to claim 1 or 2, **characterised in that** for the repeated measurement and subsequent storing of the respective new basic resistance value, a standard calibration gas (3000 p.p.m. CH₄) is used, the subsequently obtained total resistance values (Rs) being related to the respective new basic resistance value (Ro).

4. Apparatus for performing the method according to claim 1, **characterised in that** a store is provided, in which, before the measurement of the total resistance of the (semiconductor) sensor (Rs), the basic resistance value (Ro) is stored, with circuit means (microprocessor, minicomputer, logic circuit) which, on every new input of a total resistance value (Rs) of the (semiconductor) sensor, express this value as a ratio to the respective currently stored basic resistance value (Ro), and issue it for further processing.

## Revendications

1. Procédé pour le traitement des données de mesure dans le cas d'une variation de résistance pour un dispositif de détection de gaz, exploitant une concentration gazeuse à mesurer, procédé caractérisé en ce qu'au moins une fois, on détecte et on met en mémoire une valeur (Ro) de la résistance de base sans influence du gaz de mesure, et en ce que, lors de chaque mesure suivante, on calcule le rapport entre la résistance totale (Rs), résultant du composant gazeux de mesure dont la concentration est à déterminer, et la valeur de la résistance de base (Ro) et l'on poursuit le traitement avec la valeur du rapport ainsi obtenu.

2. Procédé selon la revendication 1, caractérisé en ce que la valeur de la résistance de base (Ro) est mesurée de façon répétée à des intervalles de temps prédéfinis et est mise en mémoire en tant que nouvelle valeur de résistance de base (Ro) et sert de point de départ à la formation du rapport.

3. Procédé selon la revendication 1 ou la revendication 2, caractérisé en ce que l'on utilise pour la mesure répétée et la mise en mémoire ultérieure de la nouvelle résistance de base (Ro), un gaz standard d'étalonnage ( 3 000 ppm de CH₄), les valeurs de la résistance totale (Rs) ensuite détectées étant rapportées à la valeur de la résistance de base (Ro) nouvellement détectée.

4. Dispositif pour la mise en oeuvre du procédé selon la revendication 1, caractérisé en ce qu'il est prévu une mémoire dans laquelle on stocke avant la mesure de la résistance totale du capteur (semi-conducteur) (Rs), la valeur de la résistance de base (Ro), avec des moyens de montage (microprocesseur, petit calculateur, circuit logique) qui forment le rapport et délivrent le retraitement des données, lors de chaque information nouvellement entrante au sujet d'une valeur totale de la résistance (Rs) du capteur (semi-conducteur) entre cette valeur et la valeur de la résistance de base (Ro) actuellement mise en mémoire.
